# EUROPÄISCHE PATENTANMELDUNG

(11) **EP 1 428 815 A1**
(43) Veröffentlichungstag der Anmeldung: **16.06.2004**
(21) Anmeldenummer: 03027704.0
(22) Anmeldetag: 02.12.2003
(51) Int. Cl.: C07C 211/63, C07C 57/10, C07C 63/06, A01N 33/12, A01N 37/06, A01N 31/10, A61K 7/48, A23L 3/3526

(54) **Ionische Verbindungen aus quaternären Ammoniumkationen mit Anionen von Konservierungsstoffsäuren, deren Herstellung sowie deren Verwendung zur Konservierung**

(30) Priorität: 12.12.2002 DE 10258003
(71) Anmelder: Nutrinova Nutrition Specialties & Food Ingredients GmbH, 65929 Frankfurt am Main (DE)
(72) Erfinder: Raczek, Nico N., Dr., 65779 Kelkheim (DE); Mollenkopf, Christoph, Dr., 65929 Frankfurt (DE)
(74) Vertreter: Schweitzer, Klaus, Dr.

(57) **Zusammenfassung**

Die vorliegende Erfindung betrifft ionische Verbindungen aus quaternären Ammoniumkationen mit Anionen von Konservierungsstoffsäuren, welche zur Konservierung von beispielsweise Kosmetika, Wasch- und Reinigungsmitteln, pharmazeutischen Mitteln, Bedarfsgegenständen oder als konservierend und desinfizierend wirkendes Mittel in technischen Produkten oder in Lebensmitteln, Futtermitteln u.a. eingesetzt werden können. Weiterhin betrifft die Erfindung Formulierungen, welche die erfindungsgemäßen Verbindungen und ggf. einen Stabilisator und/oder ein Formulierungshilfsmittel enthalten.

## Beschreibung

Die vorliegende Erfindung betrifft ionische Verbindungen aus quaternären Ammoniumkationen mit Anionen von Konservierungsstoffsäuren, welche zur Konservierung von beispielsweise Kosmetika, Wasch- und Reinigungsmitteln, pharmazeutischen Mitteln, Bedarfsgegenständen oder als konservierend und desinfizierend wirkendes Mittel in technischen Produkten oder in Lebensmitteln, Futtermitteln u.a. eingesetzt werden können. Weiterhin betrifft die Erfindung Formulierungen, welche die erfindungsgemäßen Verbindungen und ggf. einen Stabilisator und/oder ein Formulierungshilfsmittel enthalten.

Weltweit besteht die Tendenz, den Einsatz von Zusatzstoffen zu Lebensmitteln, Kosmetika, pharmazeutischen Mitteln, Futtermitteln etc. zu reduzieren oder komplett durch neuartige Verbindungen zu ersetzen. Insbesondere bei konservierend wirkenden Mitteln wird beispielsweise die Verwendung von halogenabspaltenden Verbindungen (z. B. Dichlorophen), Antibiotika, schwermetallhaltigen Verbindungen, schwefelhaltigen Substanzen (z. B. Schwefeldioxid), formaldehydabspaltenden Stoffen (z. B. Hexamethylentetramin), Borsäureabkömmlingen (z. B. Natriumteraborat) u.v.a.m. zunehmend eingeschränkt. Dies trifft insbesondere auf die Stoffe zu, die zur Ernährung dienen oder in direkten Kontakt mit dem menschlichen Körper kommen wie z.B. kosmetische und pharmazeutische Mittel, Bedarfsgegenstände, Wasch- und Reinigungsmittel, Lebensmittel, Futtermittel, Haustierfutter und Abfälle aus der Lebensmittelindustrie. Andere Konservierungsstoffe fallen unter die Klasse der Antibiotika (wie beispielsweise Natamycin) oder werden zukünftig in ihrer Verwendung durch gesetzliche Vorgaben weiter eingeschränkt (wie Trichloressigsäure, Schwefeldioxid und -kohlenstoff, Nitrate, Nitrite, Ester der p-Hydroxybenzoesäure, Ethylenoxid etc.).

Bei den oben genannten Konservierungsstoffen wirkt sich insbesondere die häufig nur sehr gezielte Wirksamkeit gegenüber bestimmten Mikroorganismen oder Gruppen von Mikroorganismen sehr nachteilig auf deren Verwendung aus. So wirken beispielweise Nitrite und Nitrate gut gegen bestimmte Bakterien; eine Wirkung gegen Schimmelpilze oder Hefen dagegen ist nicht festzustellen. Diphenyl wirkt gut gegen einige Hefen und Schimmelpilze, zeigt aber keine Wirkung gegen Bakterien. Auch die Wirkung der oben genannten Konservierungsstoffsäuren (Sorbinsäure, Propionsäure oder Benzoesäure) gegen Bakterien ist i.a. sehr schwach. So wirkt beispielsweise Sorbinsäure so selektiv, dass in der mikrobiologischen Diagnostik sorbinsäurehaltige Nährböden zur Züchtung von Clostridien, Milchsäurebakterien und koagulase-positive Staphylokokken verwendet werden.

Es ist bekannt, dass quaternäre Ammoniumsalze (Quats) eine antimikrobielle Wirkung haben. Die desinfizierende Wirksamkeit der Quats beschränkt sich aber auf deren bakterizide Wirkung bei höheren pH-Werten. Schimmelpilze bzw. Hefen werden nur in sehr eingeschränktem Maße bei höheren pH-Werten gehemmt. Die pH-Werte von Lebensmitteln liegen fast ausschließlich im sauren Bereich. Viele pharmazeutische Mittel, saure Reiniger oder Kosmetika werden aus Wirksamkeitsgründen sauer eingestellt. Quats können daher in diesen Bereichen nicht zur Konservierung eingesetzt werden, insbesondere wenn eine Wirkung gegen Hefen und Schimmelpilze gefordert ist.

Unterschiedliche Produkteigenschaften der zu konservierenden Erzeugnisse, insbesondere niedrige pH-Werte, führen dazu, dass es keinen allgemein optimalen Konservierungsstoff gibt, sondern dass immer produktspezifische Lösungen bei der Konservierung erforderlich sind. Solche Lösungen bedeuten aber in vielen Fällen, dass neben einem Konservierungsstoff weitere Stoffe einzusetzen sind und damit die Notwendigkeit, z. B. verschiedene Konservierungsstoffe oder synergistisch wirkende Stoffe nebeneinander in hohen Mengen zu dosieren und vorrätig zu halten. Weiterhin kommt es selbst bei gesetzlich zugelassenen Konzentrationen an Konservierungsstoffen (z. B. ca. 3750 ppm Calciumpropionat in Schnittbrot in der EU, Glutaraldehyd 1000 ppm in kosmetischen Mitteln in der EU) zu stark negativen sensorischen Veränderungen, die häufig vom Verbraucher abgelehnt werden.

Der Erfindung liegt daher die Aufgabe zugrunde, definierte Substanzen bereitzustellen, die sowohl eine keimabtötende oder keimreduzierende mikrobiostatische Wirkung haben, gleichzeitig aber auch eine Wirksamkeit gegen Schimmelpilze und Hefen in niedrigeren pH-Wertbereichen aufweisen und die leicht in der Lebensmittel-, Kosmetikoder Futterindustrie oder verwandten Industriezweigen angewendet werden können.

Unter niedrigen pH-Wertbereichen wird ein pH-Wert von < 7,0, insbesondere 2,5 - 6,0, verstanden.

Die oben genannte Aufgabe wird durch Substanzen gelöst, deren Grundkörper quaternäre Ammoniumkationen sind, die anstelle der üblichen Chlorid- oder Bromidanionen Anionen einer Konservierungsstoffsäure wie Sorbate oder Benzoate besitzen. Die Erfindung betrifft dementsprechend Verbindungen der Formel: 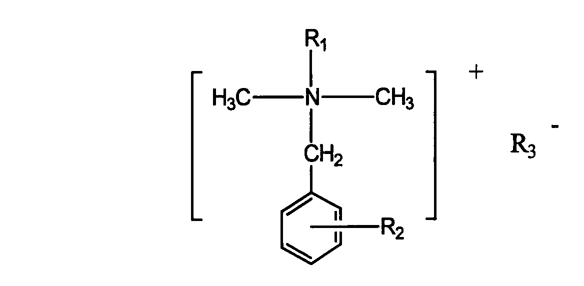 mit
R₁= C₈-C₂₀-Alkyl
R₂= H, Methyl, Ethyl, Propyl und i-Propyl
R₃= Sorboyl (trans, trans CH₃-CH=CH-CH=CH-COO), Benzoyl

Eine Möglichkeit der Herstellung solcher Verbindungen besteht in dem Austausch der Halogenidanionen eines quaternären Ammoniumhalogenids gegen Sorbat- oder Benzoatanionen, ausgehend von den handelsüblichen quaternären Ammoniumsalzen wie n-Alkyl-Dimethyl-Benzyl-ammoniumchlorid. Dazu werden beispielsweise die quaternären Ammonioumsalze in Wasser gelöst und mit einer wässrigen Lösung eines Sorbinsäure- oder Benzoesäuresalzes wie Kaliumsorbat oder Natriumbenzoat versetzt. Nach Entfernen des Wassers wird das verbleibende Reaktionsprodukt mit einem organischen Lösungsmittel wie Ethanol oder Aceton aufgenommen und so von dem anorganischen Salz der entsprechenden Gegenionen, beispielsweise Kaliumchlorid, getrennt. Man erhält die erfindungsgemäßen quaternären Ammoniumsalze als weiße wachsartige Substanzen. Alternativ kann das quaternäre Ammoniumsalz mit einer Base, zum Beispiel Natriumhydroxid, umgesetzt werden. Zu dem Reaktionsgemisch wird dann in äquimolaren Mengen Sorbinsäure bzw. Benzoesäure zugegeben. Die weitere Aufarbeitung erfolgt wie in dem obigen Herstellverfahren beschrieben.

Überraschenderweise zeigen die erfindungsgemäßen Verbindungen eine deutlich verbesserte antimikrobielle Wirkung gegen zahlreiche unterschiedliche Mikroorganismen.

Weiterhin überraschend war eine ausgezeichnete Wirkung nicht nur bei höheren pH-Werten, sondern auch bei niedrigen pH-Werten (< 7).

Der Zusatz von Synergisten oder weiteren Konservierungsstoffen bzw. pH-Wert beeinflussenden Mitteln war dazu nicht notwendig.

Die erfindungsgemäßen Verbindungen können entweder alleine als solche, insbesondere aber als Lösungen, bevorzugt als wässrige oder alkoholische Lösungen, eingesetzt werden. Sofern notwendig, können auch Formulierungen hergestellt werden, in denen die erfindungsgemäßen Verbindungen mit üblichen Additiven wie Suspendierhilfsmitteln, Verdickungsmitteln, Trägerstoffen, Filmbildnern o.ä. versetzt werden. Zur Bildung von Überzügen auf Lebensmitteln sowie zum Beschichten von Verpackungsmitteln werden zweckmäßigerweise Trägerstoffe oder Überzugs- oder Filmbildner eingesetzt. Dafür eignen sich insbesondere Stärke, Stärkeether, oxidierte oder abgebaute Stärke, Celluloseether, Alginate, Gelatine und Polyvinylalkohol.

Die erfindungsgemäßen Verbindungen lassen sich auf allen Gebieten verwenden, auf denen auch herkömmliche Konservierungsstoffe eingesetzt werden. Im folgenden werden einige Produkte beschrieben, bei denen die erfindungsgemäßen Verbindungen bevorzugt eingesetzt werden können, mit den jeweils zweckmäßigen Konzentrationen (jeweils bezogen auf die Masse des Produkts, sofern nichts anderes angegeben):

### I. Kosmetika

Unter Kosmetika sollen Mittel verstanden werden die äußerlich am Menschen zur Reinigung und Pflege oder zur Beeinflussung des Aussehens oder des Körpergeruchs angewendet werden. Dazu zählen beispielsweise:

### I.a Rasiercremes, Pflegeprodukte nach der Rasur

Die erfindungsgemäßen Verbindungen können zur Haltbarmachung von Cremes und Lotionen mit niedrigen Gehalten an Alkoholen mit etwa 0,01 - 0,2 Gew.-% eingesetzt werden.

### I.b UV-Schutzprodukte

Beispielsweise in Sonnenschutzcremes oder -lotionen sollten 0,02 - 0,3 Gew.-% der erfindungsgemäßen Verbindungen zur ausreichenden Konservierung eingesetzt werden.

### I.c Haarfärbeprodukte

Eine zweckmäßige Einsatzkonzentration für Produkte zur Färbung von Haaren, Wimpern und Augenbrauen liegt um etwa 0,1 Gew.-%, vorzugsweise 0,01 - 0,2 Gew.-%.

### I.d Feuchte Tücher

Zum Schutz vor Rekontamination und zur verlängerten Haltbarkeit können erfindungsgemäße Verbindungen beispielsweise in feuchten Babypflegetüchern in Konzentrationen zwischen 0,02 und 0,35 Gew.-% eingesetzt werden.

### I.e Handdesinfektionsmittel

Als Zusatz zu Desinfektionsmitteln der Hände können erfindungsgemäße Substanzen einzeln oder in Mischung mit anderen desinfizierend wirkenden Substanzen in Konzentrationen zwischen 0,02 und 0,5 Gew.-% eingesetzt werden.

### II. Bedarfsgegenstände

Mittel, die zur Reinigung von Gegenständen verwendet werden, die mit Lebensmitteln in Berührung kommen können oder auf diese einwirken.

### II.a Wäschenachspülmittel

Die erfindungsgemäßen Verbindungen können zur Haltbarmachung von Wäsche in Wäschenachspülmitteln wie den so genannten Weichspülern eingesetzt werden, um einen desinfizierenden Effekt auf die damit behandelte Wäsche zu erhalten. Geeignete Einsatzkonzentrationen liegen zwischen 0,005 und 0,5 Gew.-%, vorzugsweise bei 0,01 - 0,35 Gew.-%.

### II.b Geschirrwaschmittel

Mischungen aus verschiedenen Tensiden, einem eventuellem Zusatz von Stabilisatoren, Parfümölen, Farbstoffen u.a. zur gewerblichen Reinigung von Flaschen, Fässern, Gläsern etc. oder zur Anwendung in haushaltsüblichen Maschinen oder zum Geschirrwaschen mit den Händen können 0,05 - 0,3 Gew.-% an erfindungsgemäßen Substanzen enthalten.

### II.c Reinigungsmittel

Oberflächenreinigungsmittel, insbesondere solche, die zur Verminderung der Keimzahl auf Flächen in der Lebensmittel-, Kosmetik-, Pharma- oder Bedarfsgegenständeindustrie zur Anwendung kommen, werden mit 0,05 - 0,3 Gew.-% an erfindungsgemäßen Substanzen nachhaltig konserviert.

### III. Lebensmittel

### III.a Obst, Obsterzeugnisse, getrocknetes Obst, Konfitüren und Marmeladen

Eine zweckmäßige Einsatzkonzentration liegt um etwa 0,25 Gew.-%, vorzugsweise 0,05 bis 0,35 Gew.-%. Zur Oberflächenbehandlung können alkoholische oder wässrige Lösungen aufgesprüht werden, oder das Obst wird in geeignete wirkstoffhaltige Lösungen mit Gehalten bis zu 10 Gew.-% getaucht. Bei be- und verarbeiteten Produkten sollte ein Zusatz der erfindungsgemäßen Produkte zweckmäßigerweise gegen Ende des Erhitzungsprozesses erfolgen; dies trifft insbesondere auf erhitzte Produkte zu.

### III.b Gemüse, Sauergemüse, Feinkostsalate und Würzsoßen

Eine zweckmäßige Einsatzkonzentration liegt um etwa 0,25 Gew.-%, vorzugsweise 0,05 bis 0,4 Gew.-%. Zur Oberflächenbehandlung können alkoholische oder wässrige Lösungen aufgesprüht werden oder das frische Gemüse in geeignete wirkstoffhaltige Lösungen mit Gehalten von bis zu 10 Gew.-% getaucht werden. Bei be- und verarbeiteten Produkten wie Gemüsesauerkonserven wie Essiggurken, Mixed Pickles und ähnlichen und milchsauer vergorenen Gemüsen wie Sauerkraut und Oliven werden die erfindungsgemäßen Verbindungen den Aufgussflüssigkeiten üblicherweise in Mengen von 0,005 - 0,4 Gew.-%, vorzugsweise 0,05 bis 0,3 Gew.-%, zugegeben. Im gleichen Konzentrationsbereich eignen sie sich auch zur Verwendung in Feinkostsalaten, Würzsoßen und verwandten Produkten wie Senf.

### III.c Backwaren und Teige

Die erfindungsgemäßen Verbindungen lassen sich in Backwaren der verschiedensten Art, die verderbsanfällig sind, Backwarenfüllungen, teilund vorgebackenen Erzeugnissen sowie Fertigteigen problemlos einsetzen. Dafür sind je nach Produkt und gewünschter Verlängerung der Haltbarkeit Mengen bis zu 0,75 Gew.-%, vorzugsweise 0,05 bis 0,5 Gew.-%, sinnvoll.

### III.d Käse

Ein Zusatz von 0,005 - 0,5 Gew.-%, vorzugsweise 0,05 bis 0,35 Gew.-%, bezogen auf den gesamten Käselaib, reicht i. d. R. zur Konservierung von gereiften Käsen aus. Ein Zusatz zum Salzbad hält die so behandelten Käse für mehrere Wochen schimmelfrei. Vorzugsweise werden den Salzbädern von Hartkäsen täglich 1 - 10 g/l der Verbindungen, in Abhängigkeit von der Löslichkeit, zugesetzt. Eine Oberflächenbehandlung mit wässriger Lösung durch Tauchen oder Besprühen mit wässrigen Lösungen oder Suspensionen der erfindungsgemäßen Verbindungen in einer Konzentration von 10 g/l und Nachbehandlung in Intervallen von 1 bis 5 Wochen hält reifende Käse frei von Schimmel. Bei Käsen, die eine Sprühbehandlung nicht zulassen, ist auch das Auftragen einer viskos eingestellten Suspension möglich. Bei Schmelzkäse können die erfindungsgemäßen Verbindungen zusammen mit den Schmelzsalzen zugesetzt werden. Desgleichen erreicht man bei Speisequark und Sauermilcherzeugnissen durch Zusatz von 0,005 - 0,5 Gew.-%, vorzugsweise 0,05 bis 0,25 Gew.-%, der erfindungsgemäßen Verbindungen eine gute Haltbarkeitsverlängerung.

### III.e Fettemulsionen

In Fettemulsionen wie Margarine, Mayonnaise, Salatsoßen und Dressings können bis zu 0,75 Gew.-%, bevorzugt 0,05 bis 0,5 Gew.-%, eingesetzt werden. Der Zusatz erfolgt zweckmäßigerweise zur Wasserphase vor dem Emulgieren.

### III.f Fleisch- und Fischerzeugnisse

Fleisch- und Fischerzeugnisse, z. B. Pasteten und Fischmarinaden, lassen sich in den meisten Fällen mit 0,005 - 0,5 Gew.-%, vorzugsweise 0,05 bis 0,25 Gew.-%, der erfindungsgemäßen Verbindungen konservieren. Bei besonders verderbsempfindlichen Produkten wie gekochten Krustentieren können allerdings bis zu 1,0 Gew.-% erforderlich sein.

### III.g Oberflächenbehandlung und Überzüge

Die Oberflächenbehandlung von Lebensmitteln, z. B. Fleischerzeugnissen wie gereiften Würsten oder Schinken und Trockenfleisch kann in der gleichen Art und mit den gleichen Mengen wie bei gereiften Käsen erfolgen.

### IV. Verpackungen

Durch Einbringen der erfindungsgemäßen Verbindungen in Überzüge von Lebensmitteln oder technischen Produkten wie Verpackungsmaterialien wird ein bakteriostatischer Effekt erzielt. Mit etwa 1 bis 10 g der erfindungsgemäßen Verbindungen/m² wird bei Verpackungsfolien oder -papier eine Schutzwirkung gegen Bildung von Mikroorganismen, insbesondere Schimmelpilzbildung, unter der Verpackung erreicht, wenn diese im direkten Kontakt mit dem verpackten Gut stehen. Die beschichtete Verpackung kann sowohl zur Frischhaltung von frischen Lebensmitteln wie Fleisch, Fisch, Käse und Käsererzeugnissen etc. verwendet werden als auch für abgepackte Lebensmittel der verschiedensten Art wie Backwaren, Teigwaren und ,Convenience Food' im weitesten Sinne.

### V. Tierfutter und als Futtermittel geeignete andere Produkte

Der Zusatz der erfindungsgemäßen Verbindungen verlängert ebenso die Haltbarkeit von Tierfutter. Dazu gehören auch Produkte, die zur Verwendung als Futtermittel geeignet sind wie z. B. Silagen, Biertreber, Trester, Bierhefe, Destillationsschlempe und verschiedene Lebensmittelabfälle. Die erfindungsgemäßen Verbindungen können in geeigneter Pulverform trocken dem Futter zugemischt werden, vor einer weiteren Verarbeitung (z. B. Extrusion) zugegeben werden oder in Lösung aufgesprüht bzw. im Gemisch gelöst zudosiert werden. Für diese Zwecke werden Konzentrationen bis zu 2,5 Gew.-%, vorzugsweise 0,05 bis 1,5 Gew.-%, angewendet.

### VI. Arzneimittel

Zur Konservierung von wasserhaltigen pharmazeutischen Mitteln werden diesen zur Haltbarkeitsverlängerung die erfindungsgemäßen Verbindungen in einer Konzentration von 0,005 - 0,5 Gew.-%, vorzugsweise 0,05 bis 0,25 Gew.-%, zugesetzt.

### VII. Technische Produkte

Zur Konservierung von Bedarfsgegenständen, auch solchen, die zur Reinigung und Pflege von Maschinen und Geräten in der Lebensmittelindustrie vorgesehen sind, wie für mikrobiell anfällige technische Erzeugnisse können die erfindungsgemäßen Verbindungen in Konzentrationen von 0,05 - 0,5 Gew.-%, vorzugsweise 0,1 bis 0,4 Gew.-%, zugesetzt werden.

### VIII. Holz

Zur Konservierung von Holz, Holzpaletten, Schnitt- und Bruchholz können die erfindungsgemäßen Verbindungen in Konzentrationen von 0,05 bis 2,0 Gew.-% zugesetzt werden.

### IX. Farben

Zur Konservierung von Dispersionsfarben können die erfindungsgemäßen Verbindungen in Konzentrationen von 0,05 bis 2,0 Gew.-% zugesetzt werden.

Weitere Beispiele für die Verwendung der erfindungsgemäßen Verbindungen sind Produkte, die mit Kindern in Berührung kommen, wie Fingermalfarben, Knetmassen, Überzüge auf Holzspielzeug etc.

Die Erfindung wird durch die folgenden Beispiele erläutert:

### Beispiel 1

### Herstellung von n-Alkyl Dimethyl Benzyl Ammonium Sorbat

40 g n-Alkyl Dimethyl Benzyl Ammonium Chlorid (ca. 0,1 mol, n-Alkyl 60 % C₁₄, 30% C₁₆, je 5 % C₁₂ und C₁₈) wurden in Wasser gelöst und vorgelegt. Dazu wurden 15 g Kaliumsorbat in 20 g Wasser zugegeben. Bei 50 °C und 30 mbar wurde das Wasser destillativ aus dem Reaktionsgemisch entfernt. Der wachsartige Rückstand wurde in 50 ml Ethanol aufgenommen. Das Kaliumchlorid fällt als Niederschlag an und wird abgefiltert. Das Ethanol wurde aus dem Filtrat destillativ entfernt. Dieser Vorgang wurde noch zweimal wiederholt. Nach letztmaligem Entfernen des Ethanols erhält man das Produkt als weißen Rückstand.

### Beispiel 2

### Herstellung von n-Alkyl Dimethyl Ethylbenzyl Ammonium Sorbat

40 g n-Alkyl Dimethyl Ethylbenzyl Ammonium Chlorid (ca. 0,1 mol, n-Alkyl 68 % C₁₂, 32% C₁₄) wurden in Wasser gelöst und vorgelegt. Dazu wurden 15 g Kaliumsorbat in 20 g Wasser zugegeben. Bei 50 °C und 30 mbar wurde das Wasser destillativ aus dem Reaktionsgemisch entfernt. Der wachsartige Rückstand wurde in 50 ml Ethanol aufgenommen. Das Kaliumchlorid fällt als Niederschlag an und wird abgefiltert. Das Ethanol wurde aus dem Filtrat destillativ entfernt. Dieser Vorgang wurde noch zweimal wiederholt. Nach letztmaligem Entfernen des Ethanols erhält man das Produkt als weißen Rückstand.

### Beispiel 3

### Herstellung von n-Alkyl Dimethyl Benzyl Ammonium Benzoat

40 g n-Alkyl Dimethyl Benzyl Ammonium Chlorid (ca. 0,1 mol, n-Alkyl 60 % C₁₄, 30% C₁₆, je 5 % C₁₂ und C₁₈) wurden in Wasser gelöst und vorgelegt. Dazu wurden 14,4 g Natriumbenzoat in 20 g Wasser zugegeben. Bei 50 °C und 30 mbar wurde das Wasser destillativ aus dem Reaktionsgemisch entfernt. Der wachsartige Rückstand wurde in 50 ml Ethanol aufgenommen. Das Kaliumchlorid fällt als Niederschlag an und wird abgefiltert. Das Ethanol wurde aus dem Filtrat destillativ entfernt. Dieser Vorgang wurd noch zweimal wiederholt. Nach letztmaligem Entfernen des Ethanols erhält man das Produkt als weißen Rückstand.

### Beispiel 4

### Herstellung von Herstellung von n-Alkyl Dimethyl Ethylbenzyl Ammonium Benzoat

40 g n-Alkyl Dimethyl Ethylbenzyl Ammonium Chlorid (ca. 0,1 mol, n-Alkyl 68 % C₁₂, 32% C₁₄) wurden in Wasser gelöst und vorgelegt. Dazu wurden 14,4 g Natriumbenzoat in 20 g Wasser zugegeben. Bei 50 °C und 30 mbar wurde das Wasser destillativ aus dem Reaktionsgemisch entfernt. Der wachsartige Rückstand wurde in 50 ml Ethanol aufgenommen. Das Kaliumchlorid fällt als Niederschlag an und wird abgefiltert. Das Ethanol wurde aus dem Filtrat destillativ entfernt. Dieser Vorgang wurde noch zweimal wiederholt. Nach letztmaligem Entfernen des Ethanols erhält man das Produkt als weißen Rückstand.

### Beispiel 5

Das in Beispiel 2 gewonnene Produkt wurde in einem Test zur antimikrobiellen Wirksamkeit eingesetzt. Es zeigen sich sehr niedrige Mindesthemmkonzentrationen bei Mikroorganismen aus verschiedenen Bereichen (Schimmelpilze, Hefen, Bakterien).

## Patentansprüche

1. Verbindung der Formel 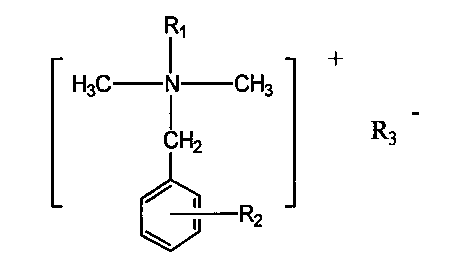 worin
R₁= C₈-C₂₀-Alkyl
R₂= H, Methyl, Ethyl, Propyl und i-Propyl
R₃= Sorboyl (trans, trans CH₃-CH=CH-CH=CH-COO), Benzoyl bedeuten.

2. Lebensmittel, **gekennzeichnet durch** einen Gehalt an einer Verbindung nach Anspruch 1.

3. Tierfutter, **gekennzeichnet durch** einen Gehalt an einer Verbindung nach Anspruch 1.

4. Kosmetikum, **gekennzeichnet durch** einen Gehalt an einer Verbindung nach Anspruch 1.

5. Technisches Produkt, **gekennzeichnet durch** einen Gehalt an einer Verbindung nach Anspruch 1.

6. Bedarfsgegenstand, **gekennzeichnet durch** einen Gehalt an einer Verbindung nach Anspruch 1.

7. Verpackung, **gekennzeichnet durch** einen Gehalt an einer Verbindung nach Anspruch 1.

8. Arzneimittel, **gekennzeichnet durch** einen Gehalt an einer Verbindung nach Anspruch 1.

9. Verwendung einer Verbindung nach Anspruch 1 zur Konservierung.

10. Verwendung einer Verbindung nach Anspruch 1 zur Oberflächenbehandlung.

11. Verwendung nach Anspruch 10 zur Oberflächenbehandlung von Obst oder Gemüse.

12. Verwendung nach Anspruch 10 in der Lebensmittelindustrie oder im Krankenhaus.

13. Mittel, enthaltend eine Verbindung nach Anspruch 1 und ein Additiv.

14. Mittel nach Anspruch 13, **dadurch gekennzeichnet, dass** das Additiv ausgewählt wird aus einer oder mehreren Substanzen der Gruppe bestehend aus: Suspendierhilfsmitteln, Verdickungsmitteln, Trägerstoffen und Filmbildnern.

15. Verfahren zur Herstellung einer Verbindung nach Anspruch 1, **dadurch gekennzeichnet, dass** man das Halogenidanion eines quaternären Ammoniumhalogenids gegen ein Sorbat- oder Benzoatanion austauscht.
